# FASCICULE DE BREVET EUROPEEN

(11) **EP 2 140 015 B1**
(45) Date de publication et mention de la délivrance du brevet: **18.04.2018**
(21) Numéro de dépôt: 08805711.2
(22) Date de dépôt: 25.04.2008
(51) Int. Cl.: C12N 15/88, C07K 14/705, C12P 21/00, A61K 38/17

(54) **FORMATION DE PROTEOLIPOSOMES CONTENANT DES PROTEINES MEMBRANAIRES, A L'AIDE D'UN SYSTEME DE SYNTHESE PROTEIQUE ACELLULAIRE**
ZELLFREIE SYNTHESE VON PROTEOLIPOSOMEN WELCHE MEMBRANPROTEINE BEINHALTEN
FORMATION OF PROTEOLIPOSOMES CONTAINING MEMBRANE PROTEINS BY MEANS OF AN ACELLULAR PROTEIN SYNTHESIS SYSTEM

(30) Priorité: 26.04.2007 FR 0754701
(43) Date de publication de la demande: 06.01.2010
(73) Titulaire: Universite Joseph Fourier, 38041 Grenoble Cédex 9 (FR)
(72) Inventeur: LENORMAND, Jean-Luc, F-38700 La Tronche (FR)
(74) Mandataire: Cabinet Laurent & Charras
(86) Numéro de dépôt international: PCT/FR2008/050757
(87) Numéro de publication internationale: WO 2008/152262

(56) Documents cités:
- WO-A1-2007/048459
- WO-A2-2007/053655
- MARQUES BRUNO ET AL: "Liposome-mediated cellular delivery of active gp91." PLOS ONE 2007, vol. 2, no. 9, 2007, page e856, XP009092806 ISSN: 1932-6203
- LIGUORI LAVINIA ET AL: "Liposomes-mediated delivery of pro-apoptotic therapeutic membrane proteins" JOURNAL OF CONTROLLED RELEASE, vol. 126, no. 3, mars 2008 (2008-03), pages 217-227, XP002506762 ISSN: 0168-3659
- KLAMMT CHRISTIAN ET AL: "Cell-free production of G protein-coupled receptors for functional and structural studies." JOURNAL OF STRUCTURAL BIOLOGY JUN 2007, vol. 158, no. 3, 23 janvier 2007 (2007-01-23), pages 482-493, XP002460324 ISSN: 1047-8477
- KLAMMT CHRISTIAN ET AL: "Evaluation of detergents for the soluble expression of alpha-helical and beta-barrel-type integral membrane proteins by a preparative scale individual cell-free expression system." THE FEBS JOURNAL DEC 2005, vol. 272, no. 23, décembre 2005 (2005-12), pages 6024-6038, XP002460325 ISSN: 1742-464X
- BERRIER CATHERINE ET AL: "Cell-free synthesis of a functional ion channel in the absence of a membrane and in the presence of detergent" BIOCHEMISTRY, vol. 43, no. 39, 5 octobre 2004 (2004-10-05), pages 12585-12591, XP002460326 ISSN: 0006-2960

## Description

### DOMAINE TECHNIQUE

La présente invention concerne une technologie efficace et originale pour produire des protéines membranaires intégrées dans la couche lipidique de liposomes, se présentant dans leur conformation native et sous forme active.

La méthode selon l'invention est basée sur la mise en oeuvre de la technique d'expression des protéines dans un système acellulaire de traduction optimisé. Lors d'une réaction en une étape, une ou plusieurs protéines membranaires recombinantes d'intérêt sont intégrées dans une bicouche lipidique définie, notamment d'origine végétale, pour produire directement des protéoliposomes actifs.

Ce procédé peut être mis en oeuvre sur des protéines membranaires d'origine variée, de structure et de fonction diverses.

Cette technologie représente un outil rapide et efficace pour produire des protéoliposomes à des fins thérapeutiques ou vaccinales.

### ETAT DE L'ART ANTERIEUR

Un intérêt grandissant pour appliquer les systèmes d'expression protéique aux biotechnologies a récemment émergé, sur la base des résultats du séquençage du génome de différentes espèces. Les applications envisageables, basées sur l'expression des protéines recombinantes, incluent la possibilité de produire rapidement de grandes quantités de protéines solubles en vue d'études structurales et fonctionnelles, d'exprimer des protéines médicament vectorisées et thérapeutiques, ainsi que le développement de microplaques à protéines pour l'étude des interactions protéine-protéine et protéine-médicament.

Des systèmes classiques de vecteur eucaryote ou bactérien sont largement utilisés pour la production des protéines recombinantes. Même si ces systèmes représentent des technologies puissantes, ils présentent un caractère limitant au niveau de plusieurs aspects. Par exemple, il est difficile dans ces systèmes d'obtenir des protéines membranaires fonctionnelles, en quantité raisonnable pour des études structurales. Il est même impossible de produire des protéines cytotoxiques.

Bien que ces systèmes de surexpression protéique soient largement utilisés, leur optimisation semble incontournable. Cette optimisation apparaît toutefois difficile à mener, sans modifier les fonctions cellulaires de l'organisme vivant hôte.

Une alternative très intéressante et attractive pour la production de protéines est l'utilisation de systèmes acellulaires de transcription-traduction (***9, 12, 13, 14***). Ces *systèmes in vitro* de synthèse de protéines utilisent essentiellement des réticulocytes de lapin, des lysats de la bactérie *Escherichia coli* ou des germes de blé.

L'un des avantages de ces *systèmes in vitro* est leur capacité à synthétiser des protéines membranaires cytotoxiques, des protéines régulatrices ou instables, qui ne peuvent pas être exprimées dans des organismes vivants, et donc dans les systèmes *in vivo* classiques. Par ailleurs, un autre avantage de ces systèmes acellulaires réside dans le fait que ce sont des systèmes complètement ouverts, dans lesquels chaque paramètre de la réaction (tel que le pH, le potentiel redox, la force ionique,...) peut être modifié, en fonction de la protéine cible à produire. En outre et dans ces systèmes, la protéine recombinante résultante représente le produit majeur de la réaction.

Actuellement, de nombreux chercheurs ont opté pour ces systèmes de synthèse comme outils pour des études structurales et fonctionnelles. Toutefois, ils ne constituent pas aujourd'hui une méthode de choix pour la production à large échelle de protéines (***5***, ***14***).

Un nombre important d'études récentes ont décrit des méthodes pour améliorer la production de protéines dans ces systèmes acellulaires. Ainsi, il a été rapporté des études reposant sur l'utilisation d'un lysat d'*E*. *coli* amélioré (***3***), sur des mélanges énergétiques modifiés (***1***), ou sur des systèmes de nouveaux réacteurs (***4***). Toutefois, aucune de ces études n'apporte la preuve directe d'une augmentation subséquente de la production de protéines.

Outre leur production, le transport des protéines jusqu'à leur site d'action reste également un point très délicat.

Parmi les systèmes de délivrance disponibles, les liposomes ont été identifiés comme de bons candidats pour la délivrance dans les cellules de macromolécules, notamment de protéines sous forme de protéoliposomes. Les liposomes présentent l'avantage d'être non cytotoxiques et peuvent délivrer et véhiculer spécifiquement une large gamme de molécules bioactives (protéines, DNA, ribozymes...). Ils protégent également les molécules de la dégradation et leur composition est aisément modifiable (***10***).

Différentes études ont déjà rapporté l'utilisation des liposomes pour le transport de protéines solubles telles que des antigènes ou des toxines, de médicaments ou d'acides nucléiques. Dans le cas des protéines membranaires, la production des protéoliposomes nécessitent une réaction en au moins deux étapes, à savoir la production de la protéine membranaire suivie de sa purification et de son incubation avec des lipides pour son intégration dans le liposome et la formation de protéoliposomes (***8***, ***16***). Dans le cas de protéines ayant tendance à s'agréger, une étape intermédiaire, supplémentaire, de dénaturation-renaturation est nécessaire. Par ailleurs et à la connaissance du Demandeur, aucune étude n'a tenté de transporter des protéines membranaires à l'aide de liposomes.

Comme déjà dit, les protéines membranaires constituent des protéines particulièrement délicates à surproduire sous forme non agrégée et active. Dans le même temps, les protéines membranaires représentent environ 30% des protéines totales d'un organisme et sont appliquées dans des processus biologiques essentiels. Ainsi, la dérégulation de leur activité biologique est une des premières réponses de la cellule aux infections bactériennes et virales, ainsi que dans les cancers et des maladies génétiques.

Cependant, les méthodes pour produire des protéines membranaires et des protéoliposomes fonctionnels sont particulièrement délicates à définir, en raison des propriétés biochimiques intrinsèques de ces protéines. Ainsi, une des difficultés majeures dans l'étude des protéines membranaires est d'obtenir des quantités suffisantes des protéines recombinantes, à partir des techniques de surexpression classiques.

Les études fonctionnelles et structurales des protéines membranaires sont donc limitées, d'une part par ce faible rendement de production, et d'autre part, par l'obtention de ces protéines majoritairement sous forme insoluble due à leur teneur élevée en acides aminés hydrophobes. Pour réaliser ces études, les protéines membranaires doivent être dénaturées et renaturées, et dans certains cas intégrées dans des liposomes. Or, ces étapes sont limitantes et longues et ne permettent pas d'obtenir des quantités importantes de protéines membranaires sous forme native. Comme preuve des difficultés rencontrées, seule la structure de 120 protéines membranaires a été résolue à ce jour.

Il existe donc un besoin persistant d'un système fiable et simple, permettant de produire des protéines membranaires en quantité importante et sous forme active.

### EXPOSE DE L'INVENTION

La présente demande décrit un procédé d'obtention de protéoliposomes contenant des protéines membranaires. Ce procédé se caractérise en ce que les protéoliposomes sont obtenus en synthétisant les protéines membranaires *in vitro* à l'aide d'un système acellulaire, et en présence de vésicules lipidiques qui sont ajoutées au milieu réactionnel.

Plus précisément, l'invention concerne un procédé d'obtention de protéoliposomes contenant des protéines membranaires. Ce procédé se caractérise en ce que l'on réalise la synthèse des protéines membranaires à l'aide d'un système de synthèse protéique acellulaire, en présence de vésicules lipidiques dans le milieu réactionnel, lesdites vésicules lipidiques étant des bicouches lipidiques se présentant sous forme de sphères d'un diamètre d'environ 100 nm et appelées SUV (Small Unilamellar Vesicles).

Les produits directement obtenus à l'issue de ce procédé sont donc des protéoliposomes contenant des protéines membranaires se présentant sous leur forme native et active.

On entend par « contenant des protéines membranaires » le fait que les protéoliposomes contiennent au moins une protéine membranaire. Il est également possible de produire et d'intégrer simultanément plusieurs protéines membranaires recombinantes, de nature différente, dans un même protéoliposome.

Les protéines membranaires, ainsi produites et conditionnées, peuvent être de toute origine : en raison des nombreuses applications visées, les protéines membranaires intégrées aux protéoliposomes selon l'invention sont avantageusement d'origine mammifères, voire humaine. Dans le cas où plusieurs protéines membranaires (de nature différente) sont contenues dans chaque protéoliposome, celles-ci peuvent être d'origine différente.

De même, il a été montré par le Demandeur que ce système fonctionnait aussi bien pour des protéines membranaires possédant un ou plusieurs domaines transmembranaires, et donc quelle que soit leur structure et leur complexité biochimique.

En outre, il est possible d'obtenir des protéines membranaires fonctionnelles, et ce quelle que soit leur fonction : canaux, récepteurs, protéines pro- ou anti-apoptotiques,...

Comme déjà dit, le procédé selon l'invention permet l'expression de protéines de diverses origines, telles que des protéines membranaires d'eucaryotes et plus particulièrement de mammifères comprenant un ou plusieurs domaines transmembranaires (récepteurs, protéines pro-apoptotiques, enzymes...), des porines d'origine bactériennes mais aussi de plantes ou de mammifères, ou des protéines d'enveloppe de virus. Des formes mutées ou tronquées de ces protéines peuvent aussi également être exprimées par ce système d'expression et être intégrées dans les vésicules lipidiques, à condition qu'elles contiennent au moins un domaine hydrophobe.

L'invention résulte d'un choix non évident du Demandeur de sélectionner, d'une part un système de synthèse protéique *in vitro,* et d'autre part la présentation des protéines membranaires sous forme de protéoliposomes. De manière remarquable, l'invention est réalisée en une seule étape, avec des résultats quantitatifs et qualitatifs très satisfaisants.

Ainsi, il a été développé et optimisé un système de surexpression acellulaire pour produire des protéines membranaires permettant à la fois d'améliorer leur synthèse sous forme soluble active, et de les intégrer directement et en une seule étape dans des liposomes pour constituer des protéoliposomes.

En pratique, une ou plusieurs protéines membranaires recombinantes sont *synthétisées in vitro* à l'aide d'un système de synthèse (transcription / traduction) des protéines acellulaire. Ces systèmes sont bien connus de l'homme du métier et sont disponibles commercialement, par exemple le système RTS (Rapid Translation System) 100HY ou 500HY commercialisé par ROCHE APPLIED SCIENCE.

Dans de tels systèmes, les protéines d'intérêt sont synthétisées à partir de plasmides dans lesquels sont clonées des séquences codant lesdites protéines ou fragments de protéines.

En général, ces séquences correspondent à un ADN complémentaire comprenant une phase de lecture ouverte codant pour une protéine membranaire contenant soit un ou plusieurs domaines transmembranaires en hélice α, soit formant une structure en feuillet β de type porine, soit contenant un domaine hydrophobe qui s'associe avec la bicouche lipidique.

Selon l'invention, il est possible de synthétiser des protéoliposomes contenant deux protéines membranaires distinctes. En pratique, chaque protéine peut être produite à partir d'un plasmide distinct. Alternativement et de manière privilégiée, les deux séquences codant les deux protéines sont placées dans un même plasmide, et avantageusement exprimées à partir d'un même promoteur.

Un système d'expression protéique acellulaire pouvant être mis en oeuvre dans le cadre de l'invention n'est pas limité à une source particulière et peut inclure des germes de blé, des lysats *d'E. coli,* des réticulocytes de lapin, ou des ovocytes de *Xenopus laevis.* De manière appropriée, l'extrait biologique est un lysat d'*E*. *coli.*

Selon l'invention, des vésicules lipidiques sont ajoutés dans le milieu réactionnel, soit au cours de la synthèse protéique, soit de manière préférentielle avant même que celle-ci ne débute. Cet ajout lipidique se fait préférentiellement à hauteur de quelques milligrammes par ml de milieu réactionnel, généralement entre 0.5 et 10 mg/ml.

Il était déjà connu de rajouter dans le milieu réactionnel des agents lipidiques, mais *a priori* de même nature que le lysat utilisé, à savoir généralement des lipides *d'E. coli,* et ce dans le but d'augmenter la solubilité des protéines néo-synthétisées. En revanche et à la connaissance du Demandeur, il n'a jamais été décrit ni même suggéré d'y ajouter des vésicules lipidiques dans le but de former des protéoliposomes.

Selon l'invention, les vésicules lipidiques correspondent à des bicouches lipidiques se présentant sous forme de sphères d'un diamètre d'environ 100 nm, préparées à l'aide de protocoles connus de l'homme du métier et appelées SUV (Small Unilamellar Vesicles) (***6***, ***15***).

Ces vésicules lipidiques peuvent être traitées à l'aide de détergents, avant leur introduction dans le milieu réactionnel.

Selon un premier mode de réalisation, les vésicules lipidiques mises en oeuvre sont d'origine naturelle, préférentiellement d'origine végétale, notamment issues d'épinards.

Dans un mode de réalisation privilégié, les vésicules lipidiques sont obtenues à partir de lipides issues de chloroplastes d'épinards. Il s'agit donc d'un mélange de lipides, essentiellement anioniques et dérivés du diacylglycérol (***2***). Dans le cadre de l'invention, il a été mis en évidence que les liposomes de thylacoïdes représentaient un matériel universel de choix dans la formation de protéoliposomes en une seule étape.

Alternativement, les vésicules lipidiques peuvent être des liposomes d'origine synthétique, c'est à dire produits à partir de lipides synthétiques.

Selon un mode de réalisation privilégié et en particulier dans le cas de lipides synthétiques, les liposomes mis en oeuvre dans le cadre de l'invention sont porteurs de molécules de polyéthylène glycol (PEG), ou de dérivés de PEG (PEG fonctionnalisé) tels que le N-carbonyl-méthoxy-polyéthylène glycol 2000.

En pratique, les molécules de PEG ou dérivés de PEG peuvent être greffés sur les lipides formant les vésicules lipidiques.

Ce mode de réalisation a un intérêt tout particulier dans la mesure où il a été montré, dans le cadre de la présente invention, que les protéoliposomes obtenus à l'issue de ce procédé sont capables de transporter et de *relarguer in vivo* les protéines membranaires d'intérêt. Ainsi, la présence de PEG ne perturbe en rien la synthèse *in vitro* des protéines membranaires ni leur intégration et leur activité dans les liposomes, tandis qu'elle augmenterait leur stabilité *in vivo.*

Avantageusement, les vésicules lipidiques mises en oeuvre dans le procédé selon l'invention ne sont pas des particules contenant des phospholipides et des apolipoprotéines (PAP).

Pour optimiser la réaction, il est possible d'ajouter dans le milieu réactionnel des composés choisis dans la liste suivante, seuls ou en combinaison : détergents (cationiques, anioniques ou zwittérioniques), chaperonnes, couples oxydo-réducteurs, polymères de carbohydrates, inhibiteurs de protéases, lipides naturels ou synthétiques. Ces derniers peuvent tout d'abord être traités avec des détergents ou utilisés directement dans la réaction. Le détergent DDM est notamment préconisé car il favorise l'insertion unidirectionnelle des protéines membranaires dans la bicouche lipidique.

A l'issue de ce procédé, les protéoliposomes représentent la population majoritaire du milieu réactionnel. Ces protéoliposomes peuvent être purifiés par tout moyen technique connu, et en particulier par passage sur un gradient discontinu de sucrose. Selon une estimation par coloration à l'argent, les fractions contenant les protéoliposomes recombinants sont pures au minimum à 90%, à l'issue de cette étape.

Les protéoliposomes ainsi obtenus contiennent les protéines membranaires dans leur conformation native, sans étape de dénaturation et/ou de renaturation. Cette méthode permet d'obtenir jusqu'à plusieurs centaines de microgrammes de protéines membranaires pures et fonctionnelles par millilitre de milieu réactionnel initial.

Selon un autre aspect, l'invention concerne donc également des protéoliposomes contenant des protéines membranaires dans leur conformation native et fonctionnelle, intégrées dans la bicouche lipidique. Il est à noter que, grâce au procédé mis en oeuvre dans le cadre de la présente invention, on obtient une population très homogène de protéoliposomes.

La nature de la bicouche lipidique formant les protéoliposomes est directement liée à celle des vésicules lipidiques ajoutées dans le milieu réactionnel. De manière avantageuse, la bicouche lipidique est donc d'origine naturelle, avantageusement d'origine végétale. Dans un mode de réalisation privilégié, le protéoliposome contient des lipides issus de chloroplastes d'épinards.

Alternativement, les protéoliposomes contiennent des lipides synthétiques.

Selon un mode de réalisation privilégié, les protéoliposomes selon l'invention sont porteurs de molécules de polyéthylène glycol (PEG), ou de dérivés de PEG (PEG fonctionnalisé) tels que le N-carbonyl-méthoxy-polyéthylène glycol 2000.

Les protéoliposomes selon l'invention peuvent faire l'objet de nombreuses applications :
- ils peuvent être utilisés directement comme mini-cellules ;
- ils peuvent servir pour tester l'activité enzymatique des protéines membranaires intégrées ou pour leur analyse structurale ;
- ils peuvent être utilisés comme vecteurs pour le relargage ciblé des protéines membranaires à la membrane plasmique ou nucléaire, à la mitochondrie ou à certaines organelles. Ces protéoliposomes peuvent donc servir comme vecteur de transduction des protéines membranaires (protéines membranaires thérapeutiques comme médicament, par exemple molécules antitumorales), mais aussi comme vecteur pour la présentation d'antigènes contenant des épitopes natifs pour le développement de vaccins.

Il peut également être envisagé de déstructurer le protéoliposome *in vitro,* éventuellement par élimination des lipides, pour récupérer les protéines membranaires en tant que telles, notamment dans l'objectif d'études structurales ou fonctionnelles.

La présente demande propose un kit permettant l'obtention de protéoliposomes contenant une ou plusieurs protéines membranaires d'intérêt. Un tel kit se compose d'au moins :
- un système de synthèse des protéines acellulaire, dans lequel va être intégré le ou les gènes codant les protéines membranaires d'intérêt ;
- des vésicules lipidiques telles que décrites ci-dessus

Plus précisément, l'invention propose un kit pour la synthèse de protéoliposomes contenant une ou plusieurs protéines membranaires d'intérêt. Un tel kit comprend un système de synthèse des protéines acellulaire et des vésicules lipidiques, lesdites vésicules lipidiques étant des bicouches lipidiques se présentant sous forme de sphères d'un diamètre d'environ 100 nm et appelées SUV (Small Unilamellar Vesicles).

La présente description met en lumière les différents avantages de la présente invention et le progrès qu'elle amène par rapport à l'art antérieur. En effet, l'obtention de protéines membranaires en quantité suffisante pour des études fonctionnelles et structurales représente un challenge depuis de très nombreuses années. Aucun des systèmes classiques de surexpression ne permettait de répondre à cette problématique. D'autre part, la haute teneur en acides aminés hydrophobes des protéines membranaires résultait dans l'obtention de protéines recombinantes insolubles, nécessitant d'être renaturées puis intégrées en plusieurs étapes dans des lipides pour étudier leur comportement.

### EXEMPLES DE REALISATION

L'invention et les avantages qui en découlent ressortiront mieux des exemples de réalisation suivants, à l'appui des figures annexées. Ceux-ci ne sont cependant en aucun cas limitatifs.

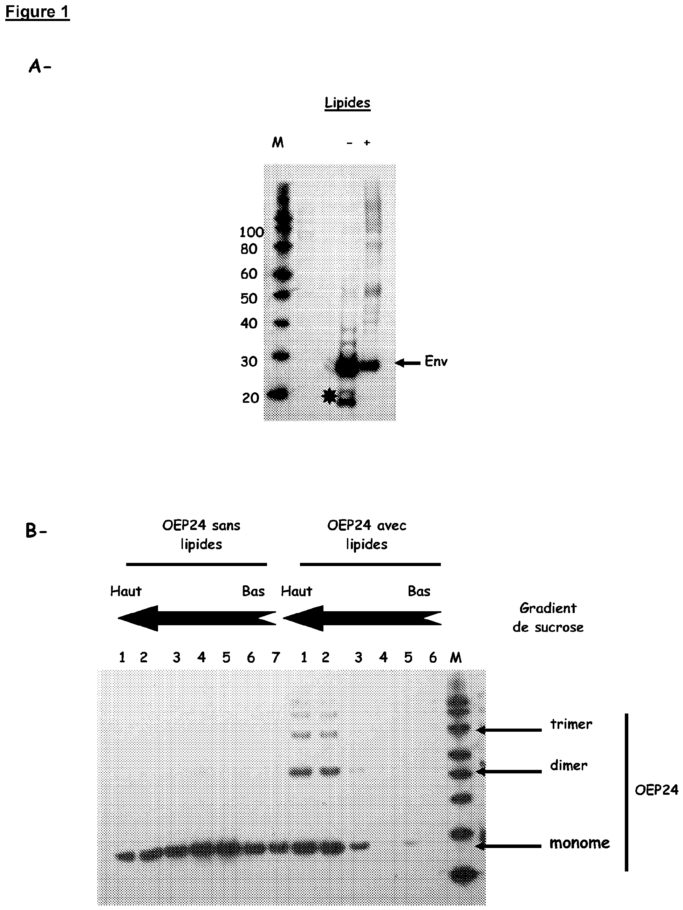

La figure 1 représente l'expression de la protéine virale Env (A) et de la porine du chloroplaste de pois OEP24 (B), en présence ou en absence de vésicules lipidiques. Les multimères de la protéine OEP24 sont indiqués par des flèches.

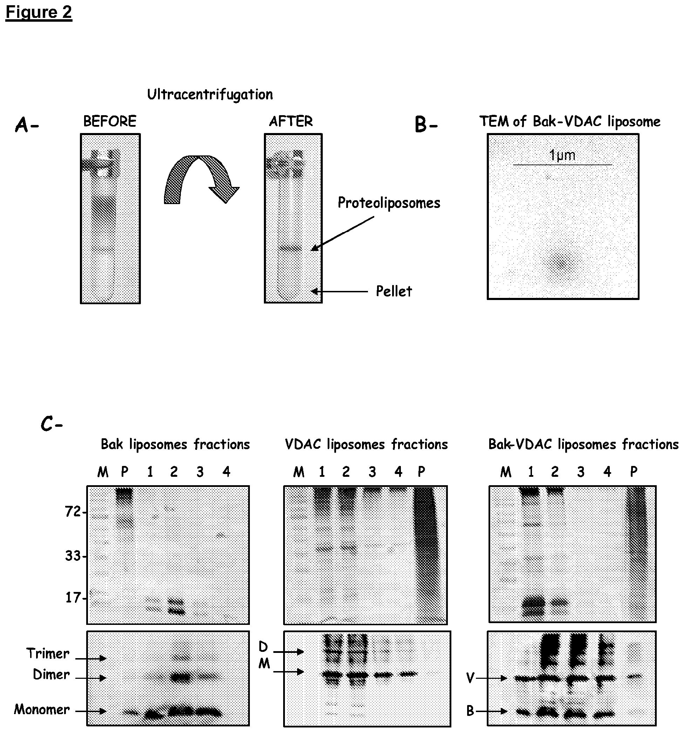

La figure 2 illustre l'analyse des protéoliposomes recombinants. (A) Purification des protéoliposomes sur gradient de sucrose. (B) Analyse par microscopie électronique des protéoliposomes recombinants purifiés. (C) Analyse sur gel SDS-PAGE et coloration au nitrate d'argent des différentes fractions des gradients de sucrose.

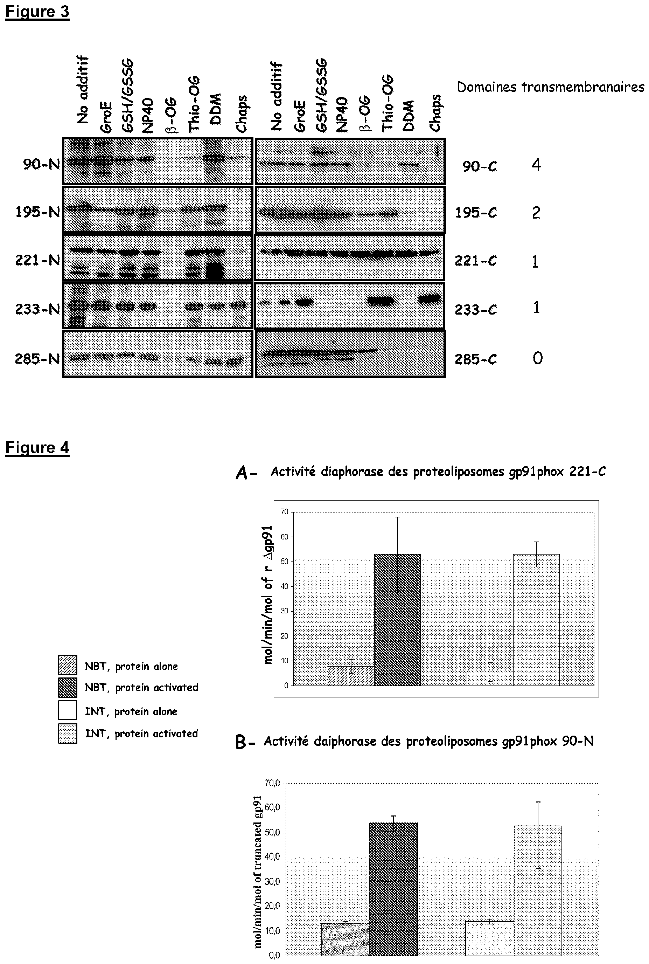

La figure 3 représente l'expression, en présence ou non de différents additifs, des différentes formes tronquées de la protéine humaine gp91-phox contenant une étiquette histidine en N- (à gauche) ou en C-terminal (à droite).
La figure 4 représente les mesures de l'activité des protéoliposomes recombinants contenant une forme tronquée de la gp91-phox à un domaine transmembranaire (A) et à 4 domaines transmembranaires (B). Les mesures des activités enzymatiques diaphorases des protéoliposomes recombinants sont réalisées en présence des substrats tels que l'INT ou le NBT et en présence ou en absence des facteurs cytosoliques.

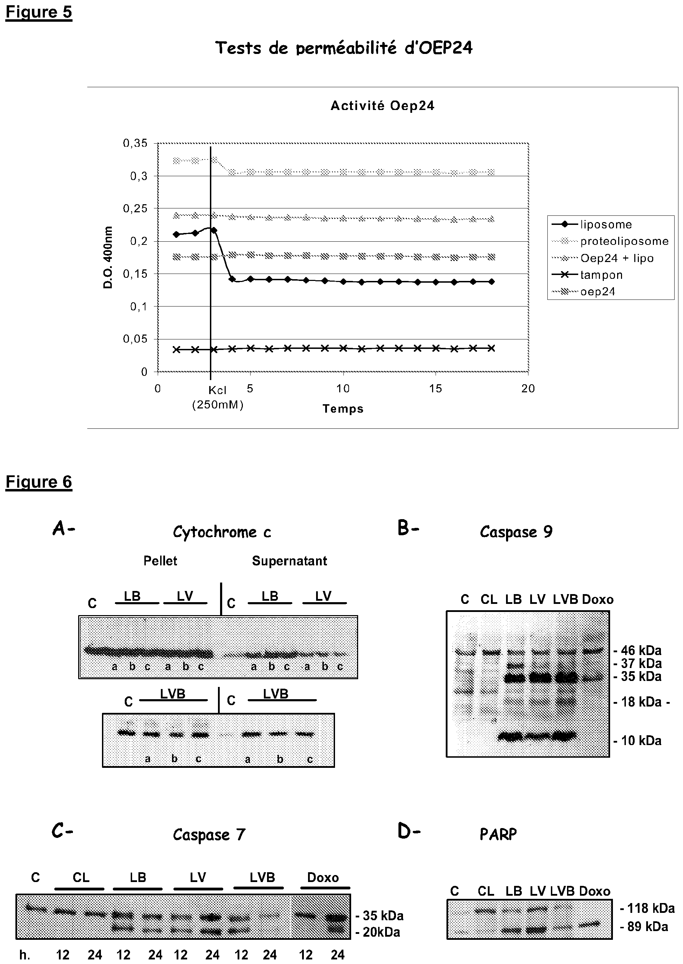

La figure 5 illustre la perméabilité des protéoliposomes contenant la protéine OEP24 au KCl. Les changements de turbidité des protéoliposomes sont mesurés à 400nm en présence de KCl.
La figure 6 représente l'activité pro-apoptotique des protéoliposomes recombinants contenant soit la protéine VDAC (LV), soit la protéine Bak (LB), soit les protéines VDAC et Bak (LVB). Les vésicules lipidiques (L) seules servent comme contrôles. L'activité pro-apoptotique des protéoliposomes recombinants est mesurée soit in vitro par étude du relargage du cytochrome c des mitochondries purifiées après interaction avec les protéoliposomes (A), soit in vivo par mesure du clivage des caspases 9 (B) et 7 (C) ou de la protéine PARP (C) après interaction et internalisation des protéoliposomes dans des cellules de carcinome de colon humain HCT116.

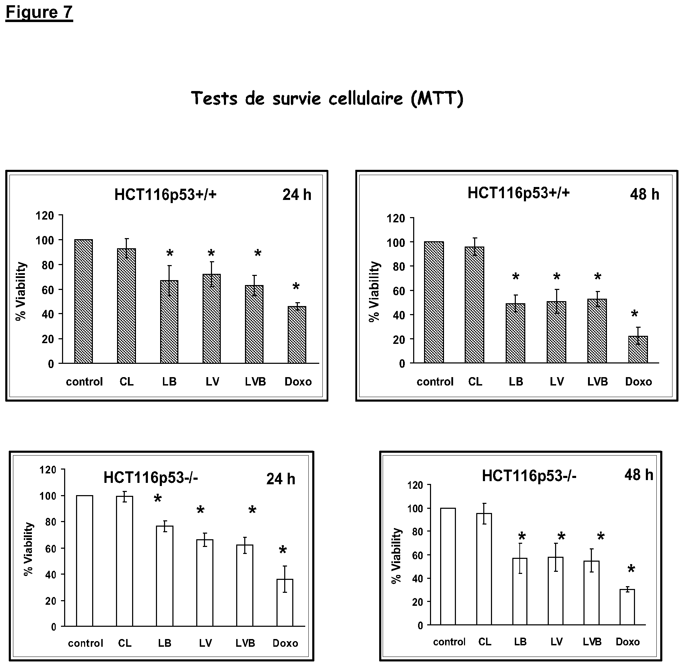

La figure 7 illustre l'activité des protéoliposomes recombinants contenant les protéines VDAC et/ou Bak (LV, LB et LVB) après internalisation dans des cellules humaines de carcinome de colon contenant la protéine p53wt (HCT116p53+/+) ou possédant une délétion du gène de la p53 (HCT116p53-/-). La survie cellulaire est mesurée 24 heures ou 48 heures après la mise en contact des protéoliposomes avec les cellules. Les liposomes seuls (CL) et la doxorubicine (Dx) servent comme contrôle négatif et positif, respectivement, de l'apoptose induite.

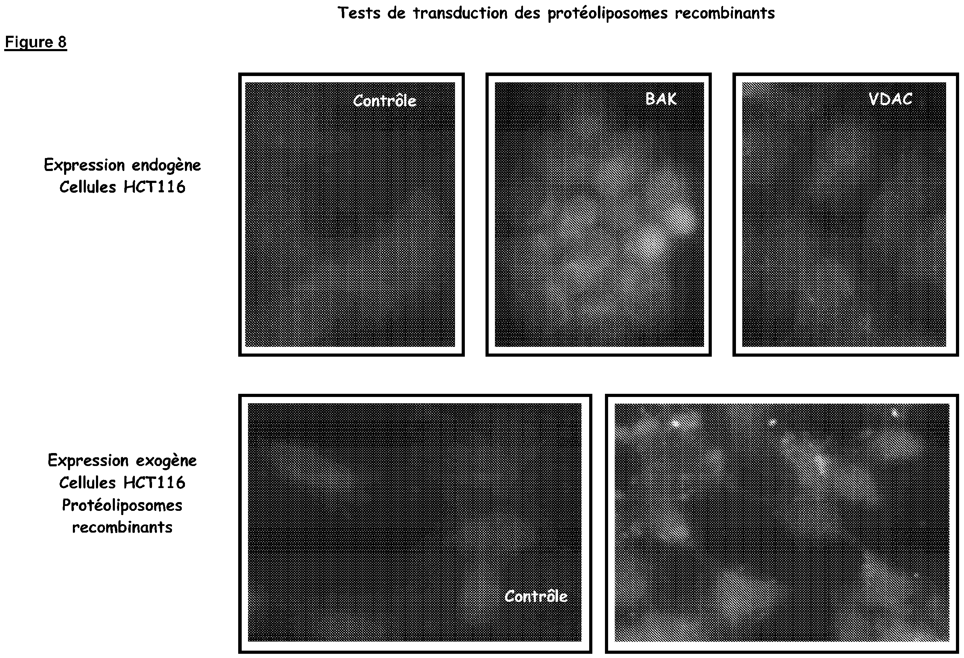

La figure 8 représente des images d'immuno fluorescence des protéines membranaires VDAC et Bak exogènes dans des cellules humaines de carcinome de colon (HCT116), après transduction et internalisation des protéoliposomes. Les protéines Bak et VDAC exogènes sont révélées par un anticorps anti-histidine et la co-localisation des protéines avec les mitochondries par coloration avec le Mitotracker. Les protéines VDAC et Bak endogènes sont révélées par des anticorps spécifiques dirigées contre les protéines VDAC1 et Bak.

### MATERIEL ET METHODES

### 1- Construction des vecteurs d'expression:

Un produit PCR contenant l'ADN complémentaire codant pour la protéine membranaire d'intérêt et comprenant une séquence promotrice et une séquence terminatrice de type T7, un RBS (Ribosome Binding Site) localisé entre 5 et 8 paires de base du premier codon méthionine peut être utilisé comme matrice pour la synthèse protéique. Toutefois, un vecteur d'expression procaryote contenant une région promotrice et une région terminatrice de type T7 ainsi qu'un RBS sera généralement préféré. Il s'agit par exemple des vecteurs pET (Novagen) ou pIVEX (Roche Appplied Science).

Dans le cas d'une co-expression de 2 protéines membranaires, chacun des ADN complémentaires codant respectivement pour ces protéines est inséré en tandem comprenant le deuxième ADN complémentaire en aval d'une seconde séquence RBS. La co-expression se fait alors sous un mode monocistronique. Pour chacune des protéines membranaires d'intérêt, une étiquette est introduite en phase soit du côté N-terminal de la protéine, soit du côté C-terminal donnant ainsi 2 constructions par ADN complémentaire. Ces étiquettes peuvent être de n'importe quelle origine (NusA, GST, MBP, etc...), et plus particulièrement une étiquette comprenant 6 ou 8 résidus histidine (6X-His ou 8X-His). La position de ces étiquettes sur la protéine peut avoir une influence importante aussi bien sur l'expression de la protéine que sur sa stabilité et sa solubilité. Chacune des constructions obtenues est vérifiée par une réaction de séquençage, afin de vérifier l'intégrité de la séquence d'ADN ainsi que sa fusion avec les étiquettes.

Les ADN complémentaires de VDAC, Bak, OEP24 et gp91-phox sont amplifiés par la technique de PCR en utilisant des couples d'amorces forward et reverse qui permettent une intégration directe et en phase après coupure par des enzymes de restriction adéquats, dans les vecteurs d'expression pIVEX2.3MCS, pIVEX2.4NdeI (Roche Applied Science) et/ou pet15b, pet30b (Novagen). Les constructions codant pour les formes tronquées de la gp91-phox utilisent la même stratégie de clonage en intégrant les sites de restriction dans les primers comme décrit ci-dessous:

### Primers forward:

5'GGAATTCCATATGGTTCGAAGACAACTGGACAGG3' pour gp91^{phox} 90 (SEQ ID 1), 5'GGAATTCCATATGAAAACCATCCGGAGGTCTTAC3' pour gp91^{phox} 195 (SEQ ID 2), 5'GGAATTCCATATGATCCATGGAGCTGAACGAA3' pour gp91^{phox} 221 (SEQ ID 3), 5'GGAATTCCATATGGCAGAGAGTTTGGCTGTG3' pour gp91^{phox} 233 (SEQ ID 4) et 5'GGAATTCCATATGTTTTGGCGATCTCAACAGA3' pour gp91^{phox} 285 (SEQ ID 5) contiennent un site NdeI du côté 5'.

### Primers reverse:

5'GCGTTACTCGAGTCATGGAAGAGACAAGTTAGAAG3' (SEQ ID 6) pour l'étiquette située en position N-terminale ou 5'GCGTTACTCGAGGAAGTTTTCCTTGTTGAAAATG3' (SEQ ID 7) pour l'étiquette située du côté C-terminal. Ces primers contiennent un site de restriction XhoI.

### 2- Préparation des vésicules lipidiques

### A - Préparations des chloroplastes intacts

Les chloroplastes sont préparés suivant la méthode décrite par Douce et Joyard (1982), avec quelques modifications. Les chloroplastes sont extraits à partir des feuilles d'épinard (*Spinacia oleracea L*.). Les feuilles sont triées, leurs nervures ôtées, puis lavées. Les feuilles ainsi obtenues sont conservées une nuit en chambre froide afin d'épuiser les réserves en amidon présentes dans les plastes. Les feuilles (environ 2 Kg pour 2 L de milieu de broyage : saccharose 0,33 M, pyrophosphate de sodium 30 mM, BSA 1 g/l, pH 7.8) sont hachées dans un broyeur (type Waring Blendor, volume 4 1) pendant 2 à 3 secondes à 4°C. Le broyat est filtré sur 4 épaisseurs de gaze et une toile à bluter de 50µm de vide de maille. Le filtrat est centrifugé à 1200 g pendant 10 min à 4°C. Les culots sont délicatement dilués avec du milieu de lavage (saccharose 0,33 M, MOPS/NaOH 20 mM, pH 7,8). Cette suspension, enrichie en chloroplastes, est filtrée sur une toile à bluter de 50µm de vide de maille. Afin d'éliminer les contaminants extraplastidiaux et les chloroplastes cassés, la suspension est déposée sur un gradient discontinu de Percoll (Percoll 40 % [v/v], saccharose 0.33 M, MOPS/NaOH 20 mM, pH 7,8 et Percoll 80 % [v/v] saccharose 0,33 M, MOPS/NaOH 20 mM, pH 7,8) et centrifugée à 3000 g pendant 20 min. Les chloroplastes intacts sont localisés à l'interface des couches de Percoll 40 et 80 % [v/v], tandis que le matériel non chloroplastique et les chloroplastes cassés restent au dessus de la couche de Percoll 40 % [v/v]. Pour éliminer le Percoll, les chloroplastes intacts sont dilués par 6 à 7 volumes de milieu de lavage et centrifugés à 4000 g pendant 5 min. Le culot est récupéré dans du tampon de lavage puis filtré sur toile à bluter de 50µm de vide de maille puis centrifugé à 3000 g pendant 5 min.

### B - Purification des thylacoïdes

La purification des thylacoïdes s'effectue suivant le protocole décrit par Douce *et al. (**2**)* avec quelques modifications. Les chloroplastes intacts sont placés dans un milieu hypotonique (MgCl₂ 4 mM, MOPS/NaOH 10 mM, pH 7,8). Le volume de milieu hypotonique est environ 10 fois supérieur à celui des chloroplastes. La concentration finale en saccharose est ainsi brutalement abaissée à moins de 0,1 M. La suspension de chloroplastes cassés est déposée sur un gradient discontinu de saccharose (saccharose 0,6 M et 0,93 M dans du tampon MgCl₂ 4 mM, MOPS/NaOH 10 mM, pH 7.8) et centrifugée à 72000 g pendant 1 h. Après centrifugation, trois fractions sont ainsi obtenues : une couche supérieure du gradient qui contient les enzymes solubles du chloroplaste (le stroma), un culot vert qui contient essentiellement des thylacoïdes, et l'enveloppe des chloroplastes localisée à l'interface des couches de saccharose 0,6 M et 0,93 M.

### C - Purification des lipides par chromatosraphie sur colonne de silice

Les compositions lipidiques de l'enveloppe du chloroplaste et des thylacoïdes étant similaires, ces dernières ont été utilisées car elles sont obtenues en plus grande quantité. Les lipides totaux sont extraits selon la méthode de Bligh & Dyer (1959), avec quelques modifications. Les thylacoïdes sont solubilisés dans du tampon de lavage ou de l'eau distillée, puis la suspension est centrifugée à 1000 g à 4°C pendant 10 min. Le culot est solubilisé dans du CHCl₃ / MeOH (1/2) auquel est rajouté un tiers de volume en CHCl₃ et un tiers d'eau distillée. La solution est conservée à 4°C pendant la nuit. La phase organique (verte foncée, contenant les lipides et les pigments) est récupérée puis évaporée à sec sous argon et solubilisée dans du CHCl₃ pur. Cette solution est déposée, pour éliminer les pigments sur une colonne de gel de silice comme décrit par Dorne *et al.* (1987). La colonne est au préalable lavée par un volume de CHCl₃ et bouchée par de laine de quartz. La silice (<325 mesh) est dissoute dans du CHCl₃ pur. Lorsque le CHCl₃ atteint le haut du gel, le dépôt est effectué. Un volume de colonne CHCl₃ est ajouté (pour éliminer les pigments) jusqu'à obtention d'un éluât translucide. Tout le CHCl₃ est alors élué et un mélange de CHCl₃/ acétone (990/10) est alors utilisé pour éluer le monogalactosyldiacylglycerol (MGDG). L'éluât est récupéré, évaporé à sec sous argon et solubilisé dans un mélange de CHCl₃/MeOH (1/2). Du méthanol est alors ajouté (élution du digalctosyldiacylglycerol (DGDG), sulfoquinovosyldiacylglycerol (SQDG), et du trigalctosyldiacylglycerol (TGDG). L'éluât est récupéré, évaporé à sec sous argon et solubilisé dans un mélange de CHCl₃/MeOH (1/2). L'échantillon est évaporé à nouveau et repris dans 3 ml de CHCl₃/MeOH (1/2) et est conservé sous argon à -20°C.

### D - Formation et purification des vésicules lipidiques

Les lipides sont évaporés sous argon et resuspendus dans de l'eau DEPC (diéthylpyrocarbonate) ou Nuclease-free. Les lipides sont soniqués (Branson sonifer 250, cycle 0,5 et amplitude 80) 3 x 1 min pour former une suspension homogène contenant des SUV (Small Unilamellar Vesicle). La suspension est filtrée sur membrane 0.2µm afin d'obtenir une suspension de vésicules lipidiques (liposomes) de taille homogène.

### 3- Expression des protéines membranaires

La synthèse des protéines membranaires s'effectue en système d'expression acellulaire. Ce système est basé sur un lysat d'*Echerichia coli* comme décrit par Spirin (***11***) ou Liguori (***7***). Les réactions s'effectuent soit en format batch (en Eppendorff ou plaques multipuits), soit en cupules (système RTS500HY de Roche Applied Science).

Les composés de la réaction sont les suivants :
- lysat d'E. *coli* (RTS system [Roche Appplied Science], RiNA system [Quiagen] ou Expressway system [Invitrogen]) ;
- le milieu énergétique ;
- l'ensemble des acides aminés sans la méthionine ;
- la méthionine ;
- les détergents non ioniques ou zwittérioniques ;
- les chaperones GroE ou DnaK ;
- les polymères de carbohydrates (NV10 [Novexin]) ;
- les inhibiteurs de protéases (e.g Protease Inhibitor Tablet [Roche Applied Science]).

Ils sont mélangés en présence de l'ADN plasmidique ou du produit PCR codant pour la protéine membranaire. Les réactions sont incubées sous agitation (de 180rpm à 990rpm) au bain Marie ou dans le Proteomaster (Roche Applied Science) de 12 heures à 48 heures à 20°C. Dans le cas de la formation en une seule étape des protéoliposomes, une fois le mélange réactionnel effectué, les vésicules lipidiques de thylacoïdes sont rajoutés sur le mélange réactionnel volume à volume et à une concentration variant de 0.6mg/ml à 10mg/ml. L'ensemble milieu réactionnel/vésicules lipidiques est ensuite homogénéisé doucement. Dans le cas d'une co-expression de 2 protéines membranaires, les 2 vecteurs d'expression (en rapport équimolaire) ou le vecteur de co-expression en mode monocistronique sous la dépendance d'un promoteur T7, sont ajoutés au volume réactionnel et incubés comme décrit ci-dessus en présence ou non de liposomes. Les réactions sont incubées comme décrites précédemment.

### 4- Purification des protéoliposomes

Les protéoliposomes produits lors de la synthèse acellulaire en batch ou en cupules sont purifiés sur un gradient discontinu de sucrose. Le mélange réactionnel est d'abord centrifugé à 13.000rpm pendant 20 min à 4°C, puis le surnageant est écarté et le culot contenant les protéoliposomes (culot de couleur verte) est resuspendu dans un volume équivalent au volume réactionnel avec du Tris-HCl 50mM, pH 7.2. Les échantillons resuspendus sont déposés entre les couches à 60% de sucrose (de 1 à 6ml de sucrose) et à 25% de sucrose (de 1 à 6ml de sucrose). L'ensemble est délicatement recouvert par une couche de sucrose à 10% (de 0.4 à 2ml de sucrose). Après centrifugation 1 heure à 200.000g à 4°C, des fractions de 0.1 à 1ml sont collectées à partir du haut du gradient et sont analysées par western blotting, coloration à l'argent et/ou au bleu de Coomassie afin de déterminer la concentration protéique finale et la pureté des protéines membranaires intégrées dans le liposome. L'activité des protéines membranaires contenue dans chacune des fractions est testée soit par réaction enzymatique (cas des formes tronquées de la gp91-phox), soit par analyse de la fonction des protéines après transduction dans des cellules de mammifères (cas des protéines Bak et VDAC). Les fractions contenant un haut degré de pureté des protéoliposomes sont groupées et testées dans des expériences de transduction dans des cellules de mammifères.

### 5- Transductions cellulaires des protéines membranaires contenues dans les protéoliposomes

Les protéoliposomes purifiés sont testés pour leurs capacités à transduire les protéines membranaires recombinantes dans des cellules de mammifères. Les protéoliposomes purifiés sont directement ajoutés à une concentration de 0.6µM à 1.5µM sur 1x10⁶ cellules et sont incubés pendant 6, 12 ou 24heures à 37°C, 5% CO2 avant analyse. La localisation cellulaire des protéines membranaires recombinantes exogènes est détectée par immuno fluorescence en utilisant des anticorps spécifiques des protéines membranaires ou un anticorps anti-histidine. Les activités des protéines membranaires recombinantes et plus particulièrement Bak et VDAC sont testées par détermination de la viabilité cellulaire (Tests MTT, Annexin V etc...), par l'activation des protéines impliquées dans l'apoptose (caspases 3, 7, 9, 8, PARP, p53 etc...) après incubation.

### RESULTATS

### 1- Expression des protéines membranaires en présence de lipides de thylacoïdes

Afin de déterminer l'influence des lipides de thylacoïdes sur la synthèse des protéines membranaires, des réactions d*'*expression *in vitro* sont réalisées en présence ou en absence de vésicules lipidiques de thylacoïdes (Figure 1A et 1B). Des réactions en batch dans un volume final de 25µl, 50µl ou 100µl sont effectuées en présence de vésicules lipidiques (liposomes) à une concentration initiale de 10mg/ml. Le volume des liposomes additionné est au moins égal à celui de la réaction de synthèse (vol/vol). La concentration finale des liposomes dans la réaction de synthèse peut varier de 0.6mg/ml à 5mg/ml. Les liposomes sont ajoutés dans le milieu réactionnel une fois le mélange réactionnel effectué. De manière intéressante, la présence des lipides n'intérfére pas avec la machinerie transcriptionnelle et traductionnelle du système d*'expression in vitro* (Figure 1). Le système d*'*expression *in vitro* basé sur un lysat d'*E*. *coli* permet la synthèse de protéines de diverses origines telles que des protéines de l'enveloppe virale (protéine Env, Figure 1A), des protéines de plantes (porine OEP24, Figure 1B), des protéines membranaires bactériennes (résultats non montrés) ou de mammifères (porine VDAC et protéine pro-apoptotique mitochondriale BAK, Figure 2C). De plus, l'addition de vésicules lipidiques a un effet positif sur la synthèse de certaines protéines membranaires et sur leurs structures, puisqu'elle permet par exemple, une protection contre la dégradation des protéines membranaires néosynthétisées (protéine Env, Figure 1A), la formation de multiméres (porine OEP24, Figures 1B et protéines mitochondriales VDAC et BAK, Figure 2C), la co-expression de protéines membranaires (Figure 2C), ou la conservation de la fonction de la protéine (protéines mitochondriales VDAC et BAK, Figures 6 et 7, et gp91-phox, Figure 4) et cela, même en absence d'additifs tels que chaperones et détergents.

### 2- Production des protéines membranaires en présence de liposomes et d'additifs

Due à la complexité structurale de certaines protéines membranaires, la synthèse sous forme soluble et active de protéines membranaires comprenant plusieurs domaines transmembranaires (tels que domaines en hélice α) nécessite l'addition de composés chimiques qui permettent de maintenir leur conformation native et leur intégration uni- ou bidirectionnelle dans la bicouche lipidique des liposomes. Différentes études sur les protéines membranaires ont démontré que l'utilisation de détergents ioniques, non ioniques ou zwittérioniques est nécessaire pour extraire et/ou resolubiliser les protéines membranaires. Ces détergents sont ensuite éliminés lors des dernières étapes de la formation des protéoliposomes soit par dialyse, par chromatographie d'exclusion, par dilution ou par addition de billes de polystyrène.

L'effet de détergents non ioniques (n-dodecyl β-D-maltoside [DDM], n-octyl β-D-glucopyranoside [β-OG], n-thiooctyl β-D-glucopyranoside [β-thioOG], nonylphenyl-polyethylene-glycol [NP40], polyoxyethylene-(23)-lauryl-ether [Brij-35], polyoxyethylene-(8)-lauryl-ether [C12E8], polyoxyethylene-sorbitan-monolaurate 20 [Tween 20], n-decyl-β-D-maltoside [DM]) et zwittérioniques (3-[(3-cholamidopropyl)dimethylammonio]-1-propanesulfonat [CHAPS], Zwittergent 3-14, Lauryldimethylamine Oxide [LDAO]) a été testé sur l'expression, la solubilité et l'intégration des protéines membranaires dans des liposomes de thylacoïdes. Des séries d'expériences ont ainsi été effectuées sur différentes formes tronquées de la protéine humaine gp91-phox en présence de détergents seuls et/ou en présence de chaperones (DNAK, GroE), d'un couple oxydo-réducteur (GSH/GSSG), d'un polymère de carbohydrate (NV10, Novexin), d'inhibiteurs de protéases (Figure 3). Tous les détergents sont ajoutés dans le milieu réactionnel à une concentration supérieure à leur concentration micellaire critique (CMC) excepté pour le n-dodecyl β-D-maltoside [DDM] qui est utilisé à une concentration proche de la CMC (0.2mM pour une CMC théorique de 0.12mM). Comme montré sur la figure 3, ces détergents ont des effets variables sur l'expression des protéines membranaires. Par exemple, le CHAPS et le β-OG ont un effet négatif sur l'expression des formes tronquées de gp91-phox excepté pour les constructions gp91^{phox} 221-C pour le β-OG et gp91^{phox} 221-C, gp91^{phox} 233-N et 233-C, gp91^{phox} 285-N pour le CHAPS. Au contraire, les détergents NP40, Thio-OG et DDM stimulent l'expression pour la plupart des formes tronquées de gp91-phhox avec un effet plus important pour les constructions contenant une étiquette hexa-histidine située en N-terminal de la protéine (Figure 3). Ces résultats suggèrent que l'addition de détergents non ioniques est compatible avec la synthèse protéique de protéines membranaires comportant un plusieurs domaines transmembranaires en hélice α et peuvent être ajoutés dans la réaction de synthèse pour la formation de protéoliposomes.

Des expériences de synthèse de protéines membranaires comportant un ou plusieurs domaines transmembranaires en hélice α ont été réalisées en présence de détergents et de liposomes pour former des protéoliposomes. Les résultats précédents ont démontré que le détergent DDM à une concentration proche de sa CMC, stimulait majoritairement l'expression et la solubilité des différentes formes tronquées de la protéine gp91-phox. Le détergent DDM a donc été préférentiellement choisi dans les expériences de formation des protéoliposomes. D'autre part, il a été démontré que grâce à ses propriétés biochimiques, le DDM favorisait l'intégration unidirectionnelle en sens des protéines membranaires dans les bicouches lipidiques. Les formes tronquées de la protéine gp91-phox contenant un ou plusieurs domaines transmembranaires de la protéine ont été produites en présence du DDM et de vésicules lipidiques. Les protéoliposomes sont ensuite purifiés sur gradient de sucrose et l'activité diaphorase des fractions contenant les protéoliposomes purifiés a été testée en présence de NBT ou de INT.

### 3- Activité fonctionnelle des protéoliposomes synthétisés dans un système in vitro

L'activité enzymatique des protéoéliposomes contenant les différentes formes tronquées de la gp91-phox a été réalisée en présence de 2 substrats comme accepteurs d'électrons: le NitroBlueTetrazolium (NBT) et l'IodoNitroTetrazolium (INT). A la différence des protéines tronquées solubles gp91-phox, les protéoliposomes possèdent une activité enzymatique NADPH et FAD dépendantes sans l'addition de facteurs cytosoliques et d'acide arachidonique (Figure 4). Par exemple, l'activité diaphorase du protéoliposome contenant la protéine gp91-phox 221C en présence des 2 co-facteurs possède une activité basale de 7.8 mol/min/mol pour la réduction du NBT et de 5.5 mol/min/mol pour l'INT (Figure 4A et 4B). Ces valeurs sont proches de celles de la protéine soluble et indiquent que le protéoliposome est pleinement fonctionnel. L'incubation de 10nmoles de protéoliposomes recombinants contenant la gp91phox-221C avec des extraits cytosoliques et de l'acide arachidonique entraîne une augmentation de l'activité réductase de la protéine par un facteur 7 pour le NBT et 10 pour l'INT (Figure 4A et 4B). Ces résultats indiquent donc que les protéines membranaires recombinantes contenues dans les vésicules lipidiques possèdent des activités enzymatiques basales qui peuvent être stimulées par l'addition de facteurs cytosoliques et d'acide arachidonique.

Afin de déterminer l'activité des protéoliposomes contenant la porine OEP24, les tests d'activité sont réalisés par des mesures sur les différences d'absorption à 400nm (Figure 5). Les vésicules lipidiques contenant ou non OEP24 sont tout d'abord équilibrés dans une solution contenant 10 mM de Tris-Hepes, pH 7.0 et 10 mM de KCl. L'absorbance à 400 nm est ensuite mesurée pendant 15 sec puis l'osmoticum (KCl 250 mM final) est rajouté dans le milieu. Les variations dans l'absorbance sont mesurées périodiquement pendant 2 minutes. En présence de KCl, les vésicules lipidiques vides (sans OEP24) présentent une variation dans l'absorption à 400nm due à un choc osmotique. Les protéoliposomes contenant la protéine membranaire OEP24 ne présentent pas de variation de l'absorption à 400nm indiquant que les vésicules lipidiques sont perméables à l'osmoticum ajouté (Figure 5). Ces résultats démontrent que la protéine OEP24 est pleinement active et est intégrée majoritairement en orientation sens dans la bicouche lipidique.

Les protéoliposomes contenant les protéines pro-apoptotiques Bak et/ou VDAC sont mis en présence de mitochondries purifiées afin de mesurer le relargage du cytochrome c. Comme montré dans la Figure 6A, les protéoliposomes contenant les protéines VDAC et/ou Bak induisent un relargage massif du cytochrome c dans le surnageant en comparaison avec les liposomes contrôles. Ces résultats indiquent que les protéoliposomes recombinants interagissent directement avec la membrane externe des mitochondries et induisent le relargage du cytochrome c. D'autre part, l'ensemble de ces résultats confirment que la synthèse en une seule étape permet d'obtenir rapidement des préparations de protéoliposomes pleinement fonctionnels contenant des protéines membranaires de diverses complexités en orientation positive.

### 4- Exemples d'applications thérapeutiques des protéoliposomes formés in vitro

Un exemple d'application est illustré dans les Figures 6B, 6C, 6D, 7 et 8. Afin de mesurer l'activité proapoptotique *in vivo* des protéines VDAC et Bak insérés dans les vésicules lipidiques, des cellules de carcinome de colon humain (HCT116) sont incubées en présence des protéoliposomes recombinants pendant 24 et 48 heures. La localisation des protéines membranaires exogènes est confirmée par immunofluorescence (Figure 8B) et l'activité proapoptotique est mesurée soit par la stimulation de la voie des caspases (Figure 6B, 6C et 6D), soit par mesure de la viabilité cellulaire (Figure 7). L'ensemble de ces résultats indiquent que les protéoliposomes recombinants sont capables de relarguer spécifiquement les protéines membranaires d'intérêt thérapeutique à la membrane externe des mitochondries et d'induire l'apoptose dans les cellules cibles. D'une manière identique, les protéoliposomes sont capables de relarguer les formes tronquées de la gp91-phox à la membrane plasmique (résultats non montrés).

En conclusion, la formation en une seule étape de protéoliposomes recombinants comprenant une ou plusieurs protéines membranaires peut représenter une méthode de choix pour le développement de vecteurs pour le relargage de protéines membranaires à visée thérapeutiques ou antigéniques, pour le criblage de drogues chimiques qui interagissent avec les protéines membranaires, pour les études structurales et fonctionnelles des protéines membranaires et pour la mise en place de kits pour la synthèse rapide de protéines membranaires.

### REFERENCES

**(1)** Calhoun KA, Swartz JR. (2005) Energizing cell-free protein synthesis with glucose metabolism. Biotechnol Bioeng, 90(5), 606-613.
**(2)** Douce R, Holtz RB, Benson AA. (1973) Isolation and properties of the envelope of spinach chloroplasts. J Biol Chem. 248(20):7215-22.
**(3)** Kim DM, Swartz JR. (2000) Prolonging cell-free protein synthesis by selective reagent additions. Biotechnol Prog, 16(3), 385-390.
**(4)** Kim TW, Kim DM, Choi CY. (2006) Rapid production of milligram quantities of proteins in a batch cell-free protein synthesis system. J Biotechnol, 124(2):373-80.
**(5)** Lamla T., Mammeri K. and Erdmann VA (2001) The cell-free protein biosynthesis: applications and analysis of the system. Acta Biochim Pol. 48(2):453-65.
**(6)** Lasic DD (1997) in Liposomes in gene delivery. CRC Press LLC
**(7)** Liguori L, Marques B, Villegas-Mendez A, Rothe R and JL Lenormand (2007), Production of membrane proteins using cell-expression systems. Expert Rev Proteomics. 4(1):79-90.
**(8)** Rigaud JL, (2002) Membrane proteins: functional and structurale studies using reconstitued proteoliposomes and 2-D crystals. Braz. J. Med. Biol. Res. 35(7):753-766.
**(9)** Ryabova LA, Morozov Iyu and Spirin AS (1998) Continuous-flow cell-free translation, transcription-translation, and replication-translation systems. Methods Mol Biol. 77:179-93.
**(10)** Service RF, (2005) Nanotechnology takes aim at cancer. Science 310(5751), 1132-1134.
**(11)** Spirin AS (2002): in Cell-free translations systems. Springer, 3-20.
**(12)** Spirin AS (2004) High-throughput cell-free systems for synthesis of functionally active proteins. Trends Biotechnol 22: 538-545
**(13)** Swartz JR (2003) Cell-Free Protein Expression: A Springer press, Springer Berlin, Heidelberg, New York.
**(14)** Swartz JR (2006) Developing cell-free biology for industrial applications. J Ind Microbiol Biotechnol. 33(7):476-85
**(15)** Templeton NS, Lasic DD, Frederick PM, Strey HH, Roberts DD and Pavlakis GN (1997) Improved DNA:liposome complexes for increased systemic delivery and gene expression. Nature Biotech. 15(7): 647-652.
**(16)** Klammt C, Schwarz D, Eifler N, Engel A, Piehler J, Haase W, Hahn S, Dötsch V, Bernhard F (2007) Cell-free production of G protein-coupled receptors for functional and structural studies. J Struct Biol. 158(3):482-93.

## Revendications

1. Procédé d'obtention de protéoliposomes contenant des protéines membranaires ***caractérisé* en ce que** l'on réalise la synthèse des protéines membranaires à l'aide d'un système de synthèse protéique acellulaire, en présence de vésicules lipidiques dans le milieu réactionnel, lesdites vésicules lipidiques étant des bicouches lipidiques se présentant sous forme de sphères d'un diamètre d'environ 100 nm et appelées SUV (Small Unilamellar Vesicles).

2. Procédé selon la revendication 1, ***caractérisé* en ce que** les vésicules lipidiques sont porteuses de molécules de Polyéthylène Glycol (PEG) ou de ses dérivés.

3. Procédé selon la revendication 2, ***caractérisé* en ce que** les molécules de Polyéthylène Glycol (PEG) ou de ses dérivés sont greffées sur des lipides synthétiques.

4. Procédé selon l'une des revendications précédentes, ***caractérisé* en ce que** les vésicules lipidiques contiennent des lipides d'origine végétale.

5. Procédé selon la revendication 4, ***caractérisé* en ce que** les lipides d'origine végétale sont issus de chloroplastes d'épinards.

6. Protéoliposomes contenant des protéines membranaires dans leur conformation native intégrées dans une bicouche lipidique, obtenus à l'aide du procédé selon l'une des revendications 1 à 5.

7. Protéoliposomes selon la revendication 6, ***caractérisés* en ce qu'**ils sont porteurs de molécules de Polyéthylène Glycol (PEG) ou de ses dérivés.

8. Protéoliposomes selon la revendication 7, ***caractérisés* en ce que** les molécules de Polyéthylène Glycol (PEG) ou de ses dérivés sont greffées sur des lipides synthétiques.

9. Protéoliposomes selon l'une des revendications 6 à 8, ***caractérisés* en ce que** les lipides formant la bicouche lipidique sont d'origine végétale.

10. Protéoliposomes selon la revendication 9, ***caractérisés* en ce que** les lipides formant la bicouche lipidiques sont issus de chloroplastes d'épinards.

11. Protéoliposomes selon l'une des revendications 6 à 10, ***caractérisés* en ce que** les protéines membranaires sont des protéines de mammifères.

12. Protéoliposomes selon la revendication 11, ***caractérisés* en ce que** les protéines membranaires sont des protéines d'origine humaine.

13. Protéoliposomes selon l'une des revendications 6 à 12 pour leur utilisation comme médicament ou comme vaccin.

14. Utilisation *in vitro* de protéoliposomes selon l'une des revendications 6 à 12 comme mini-cellule.

15. Kit pour la mise en oeuvre du procédé selon l'une des revendications 1 à 5 ou pour la synthèse de protéoliposomes selon l'une des revendications 6 à 12 comprenant un système de synthèse des protéines acellulaire et des vésicules lipidiques, lesdites vésicules lipidiques étant des bicouches lipidiques se présentant sous forme de sphères d'un diamètre d'environ 100 nm et appelées SUV (Small Unilamellar Vesicles).

## Patentansprüche

1. Verfahren zum Erhalten von Proteoliposomen, die Membranproteine enthalten, **dadurch gekennzeichnet, dass** die Synthese der Membranproteine mit Hilfe eines azellulären Proteinsynthesesystems durchgeführt wird, in Anwesenheit von Lipidvisikeln im Reaktionsmedium, diese Lipidvesikel sind Lipid-Doppelschichten und haben die Form von Kugeln, mit einem Durchmesser von ungefähr 100 nm und werden SUV (Small Unilamellar Vesicles) genannt.

2. Verfahren gemäß Anspruch 1, **dadurch gekennzeichnet, dass** die Lipidvesikel Träger von Polyethylenglykol-(PEG)-Molekülen oder ihren Derivaten sind.

3. Verfahren gemäß Anspruch 2, **dadurch gekennzeichnet, dass** die Polyethylenglykol-(PEG)-Moleküle oder ihre Derivate auf synthetische Lipide aufgepfropft werden.

4. Verfahren gemäß einem der vorstehenden Ansprüche, **dadurch gekennzeichnet, dass** die Lipidvesikel Lipide pflanzlicher Herkunft enthalten.

5. Verfahren gemäß Anspruch 4, **dadurch gekennzeichnet, dass** die Lipide pflanzlicher Herkunft aus Spinatchloroplasten stammen.

6. Proteoliposomen, die Membranproteine enthalten, in ihrer nativen Konformation, integriert in eine Lipid-Doppelschicht, erhalten mittels des Verfahrens nach irgendeinem der vorstehenden Ansprüche 1 bis 5.

7. Proteoliposome gemäß Anspruch 6, **dadurch gekennzeichnet, dass** sie Träger von Polyethylenglykol-(PEG)-Molekülen oder ihren Derivaten sind.

8. Proteoliposome gemäß Anspruch 7, **dadurch gekennzeichnet, dass** die Polyethylenglykol-(PEG)-Moleküle oder ihre Derivate auf synthetische Lipide aufgepfropft werden.

9. Proteoliposome gemäß einem der vorstehenden Ansprüche 6 bis 8, **dadurch gekennzeichnet, dass** die Lipide, die die Lipid-Doppelschicht bilden, pflanzlicher Herkunft sind.

10. Proteoliposome gemäß Anspruch 9, **dadurch gekennzeichnet, dass** die Lipide, die die Lipid-Doppelschicht bilden, aus Spinatchloroplasten stammen.

11. Proteoliposome gemäß einem der vorstehenden Ansprüche 6 bis 10, **dadurch gekennzeichnet, dass** die Membranproteine Säugetierproteine sind.

12. Proteoliposome gemäß Anspruch 11, **dadurch gekennzeichnet, dass** die Membranproteine Proteine menschlichen Ursprungs sind.

13. Proteoliposome gemäß einem der vorstehenden Ansprüche 6 bis 12, zur Verwendung als Medikament oder als Impfstoff.

14. In vitro-Verwendung von Proteoliposomen nach irgendeinem der vorstehenden Ansprüche 6 bis 12 als Mini-Zelle.

15. Set zum Einsatz des Verfahrens nach irgendeinem der vorstehenden Ansprüche 1 bis 5 oder zur Proteoliposomsynthese nach irgendeinem der vorstehenden Ansprüche 6 bis 12, mit einem azellulären Proteinsynthesesystem und Lipidvisikeln, diese Lipidvesikel sind Lipid-Doppelschichten und haben die Form von Kugeln, mit einem Durchmesser von ungefähr 100 nm und werden SUV (Small Unilamellar Vesicles) genannt.

## Claims

1. Process for obtaining proteoliposomes containing membrane proteins ***characterized* in that** the synthesis of the membrane proteins is made using a cell-free system of protein synthesis, in the presence of lipid vesicles in the reaction medium, wherein the lipid vesicles are lipid bilayers in the form of spheres with a diameter of approximately 100 nm and known as SUV (Small Unilamellar Vesicles).

2. Process according to claim 1, ***characterized* in that** the lipid vesicles bear polyethylene glycol (PEG) molecules or its derivatives.

3. Process according to claim 2, ***characterized* in that** the polyethylene glycol (PEG) molecules or its derivatives are grafted onto synthetic lipids.

4. Process according to any of the preceding claims, ***characterized* in that** the lipid vesicles contain lipids of plant origin.

5. Process according to claim 4, ***characterized* in that** the lipids of plant origin are from spinach chloroplasts.

6. Proteoliposomes containing membrane proteins in their native conformation integrated into a lipid bilayer, obtained using the process according to one of claims 1 to 5.

7. Proteoliposomes according to claim 6, ***characterized* in that** they bear polyethylene glycol (PEG) molecules or its derivatives.

8. Proteoliposomes according to claim 7, ***characterized* in that** the polyethylene glycol (PEG) molecules or its derivatives are grafted onto synthetic lipids.

9. Proteoliposomes according to claims 6 to 8, ***characterized* in that** the lipids forming the lipid bilayer are of plant origin.

10. Proteoliposomes according to claim 9, ***characterized* in that** the lipids forming the lipid bilayer are from spinach chloroplasts.

11. Proteoliposomes according to claims 6 to 10, ***characterized* in that** the membrane proteins are mammalian proteins.

12. Proteoliposomes according to claim 11, ***characterized* in that** the membrane proteins are of human origin.

13. Proteoliposomes according to one of claims 6 to 12 for their use as a medicine or as a vaccine.

14. *In vitro* use of proteoliposomes according to one of claims 6 to 12 as mini-cells.

15. Kit for carrying out the process according to one of the claims 1 to 5 or for the synthesis of proteoliposomes according to one of the claims 6 to 12 including a cell-free system of protein synthesis and lipid vesicles, wherein the lipid vesicles are lipid bilayers in the form of spheres with a diameter of approximately 100 nm and known as SUV (Small Unilamellar Vesicles).


### SEQUENCE LISTING

<110> UNIVERSITE JOSEPH FOURIER
<120> FORMATION DE PROTEOLIPOSOMES CONTENANT DES PROTEINES MEMBRANAIRES, A L'AIDE D'UN SYSTEME DE SYNTHESE PROTEIQUE ACELLULAIRE
<130> U19-B-24219PCT
<150> FR07.54701
   <151> 2007-04-26
<160> 7
<170> PatentIn version 3.1
<210> 1
   <211> 34
   <212> DNA
   <213> artificial sequence
<220>
   <223> gp91phox 90
<400> 1
   ggaattccat atggttcgaa gacaactgga cagg 34
<210> 2
   <211> 34
   <212> DNA
   <213> artificial sequence
<220>
   <223> gp91phox 195
<400> 2
   ggaattccat atgaaaacca tccggaggtc ttac 34
<210> 3
   <211> 32
   <212> DNA
   <213> artificial sequence
<220>
   <223> gp91phox 221
<400> 3
   ggaattccat atgatccatg gagctgaacg aa 32
<210> 4
   <211> 31
   <212> DNA
   <213> artificial sequence
<220>
   <223> gp91phox 233
<400> 4
   ggaattccat atggcagaga gtttggctgt g 31
<210> 5
   <211> 32
   <212> DNA
   <213> artificial sequence
<220>
   <223> gp91phox 285
<400> 5
   ggaattccat atgttttggc gatctcaaca ga 32
<210> 6
   <211> 35
   <212> DNA
   <213> artificial sequence
<220>
   <223> primer reverse Nterm
<400> 6
   gcgttactcg agtcatggaa gagacaagtt agaag 35
<210> 7
   <211> 34
   <212> DNA
   <213> artificial sequence
<220>
   <223> primer reverse Cterm
<400> 7
   gcgttactcg aggaagtttt ccttgttgaa aatg 34